# NEUE EUROPÄISCHE PATENTSCHRIFT

(11) **EP 1 399 105 B2**
(45) Veröffentlichungstag und Bekanntmachung des Hinweises auf die Entscheidung über den Einspruch: **05.09.2018**
(45) Hinweis auf die Patenterteilung: 16.12.2009
(21) Anmeldenummer: 02747374.3
(22) Anmeldetag: 12.06.2002
(51) Int. Cl.: A61J 3/07

(54) **VERFAHREN ZUR REINIGUNG VON HARTGELATINEKAPSELN**
METHOD FOR CLEANING HARD GELATINE CAPSULES
PROCEDE POUR NETTOYER DES CAPSULES DE GELATINE DURE

(30) Priorität: 13.06.2001 DE 10128779
(43) Veröffentlichungstag der Anmeldung: 24.03.2004
(73) Patentinhaber: Boehringer Ingelheim Pharma GmbH & Co. KG, 55216 Ingelheim am Rhein (DE)
(72) Erfinder: WACHTEL, Herbert, 55411 BINGEN (DE); FREUDENBERGER, Volker, 55270 SCHWABENHEIM (DE); SCHMIDT-JOERG, Petra, 55218 INGELHEIM (DE)
(86) Internationale Anmeldenummer: PCT/EP2002/006423
(87) Internationale Veröffentlichungsnummer: WO 2002/100319

(56) Entgegenhaltungen:
- WO-A-00/47200
- WO-A-02/30389
- WO-A-96/01105
- WO-A1-94/06498
- CA-A1- 2 449 217
- DE-A1- 1 491 715
- GB-A- 697 723
- GB-A- 1 122 284
- US-A- 3 653 500
- US-A- 3 676 958
- US-A- 3 858 583
- US-A- 3 860 618
- US-A- 3 948 264
- US-A- 4 069 819
- US-A- 4 590 206
- US-A- 4 889 114
- US-A- 5 641 510
- AULTON M.E.: 'Pharmaceutics: The Science of Dosage Form Design', 1988, CHURCHILL LIVINGSTONE, EDINBURGH Seiten 680 - 681
- BARNES P.J. ET AL: 'The Role of Anticholinergics in Chronic Obstructive Pulmonary Disease and Chronic Asthma', 1997, GARDINER-CALDWELL COMMUNICATION LTD Seiten 126 - 136

## Beschreibung

Die Erfindung betrifft ein Verfahren zur Reinigung der Innenwand von Hartgelatinekapseln.

### Hintergrund der Erfindung

Bedingt durch die Herstellung von Hartgelatinekapseln ist die Innenwand dieser Kapseln mit Schmier- und/oder Trennmitteln belegt. Dies führt zu einer erhöhten Haftung von Pulver an deren Innenwänden, welches bei Kapseln zur Inhalation nicht dem Patienten zugeführt werden kann. Zusätzlich unterliegen die Parameter "ausgebrachte Masse" und "inhalierbarer Anteil" einer hohen Schwankungsbreite. WO 96/01105 beschreibt ein Verfahren umfassend die Belegung der Kapselinnenwand mit Pulver zur Bildung einer Haftungsschicht auf der Schmier- und Trennmittelschicht und anschließender Entfernung des überschüssigen Pulvers. Die Verwendung der so vorbereiteten Kapseln im Pulverinhalator führt zu erhöhter Ausbringung der Wirkstoffdosis.
Aus dem Stand der Technik ist ebenfalls bekannt, daß spezielle Kapseln, beispielsweise CONI-SNAP^{®} -Kapseln (Fa. Capsugel), welche einen verringerten Schmier- bzw. Trennmittelbelag aufweisen, für Inhalationszwecke eingesetzt werden. Dieser Belag kann mit Lösungsmitteln entfernt werden. Die Reinigung mit Lösungsmitteln ist im technischen Maßstab jedoch sehr aufwendig und daher nur bedingt geeignet. Zudem ist eine anschließende Kontrolle des Gehalts an Restlösungsmittel erforderlich.
Es ist die Aufgabe der vorliegenden Erfindung ein Verfahren bereitzustellen, welches die einfache Reinigung der Innenwand von Hartgelatinekapseln zur Verwendung in der Inhalationstherapie ermöglicht.

### Detaillierte Beschreibung der Erfindung

Überraschenderweise wurde gefunden, daß bei der Verwendung von geeigneten Pulvern als Reinigungsmittel ohne Lösungsmittelzusatz der Schmier- bzw. Trennmittelbelag von Kapselinnenwänden auf einfache Weise entfernt wird.

Die Erfindung betrifft daher ein im Labormaßstab und ebenso technisch anwendbares Verfahren zur Reinigung der Innenwand von Hartgelatinekapseln zur Verwendung in der Inhalationstherapie, wobei die Kapseln durch Schütteln in einem Freifallmischer oder auf einem Rütteltisch mit einer Pulverformulierung gereinigt werden.
Für das erfindungsgemäße Verfahren geeignete Kapseln sind in der Regel teilbare Hartgelatinekapseln, bestehend aus Ober- und Unterteil, wobei nach dem Füllen des Unterteils das Oberteil aufgesetzt und die Kapsel so verschlossen wird. Vorzugsweise geeignete Kapseln sind Hartgelatine Steckkapseln mit Verschluß, bevorzugt polyethylenglycolfreie Hartgelatine Kapseln, besonders bevorzugt CONI-SNAP® Kapseln der Größe 3 (Hersteller: Capsugel, Division of Warner Lambert N.V., Belgien).

Bevorzugt ist ein Verfahren, worin die Pulverformulierung pharmazeutisch verträglich ist. Die Pulverformulierung kann ein oder mehrere Bestandteile enthalten. Pharmazeutisch verträgliche Bestandteile sind beispielsweise Lactose, Lactose Monohydrat, Glucose, Saccharose, Mannit, und Sorbit.
Besonders bevorzugt ist ein Verfahren, worin die Pulverformulierung einen Bestandteil der Wirkstoffformulierung darstellt. Für die Pulverformulierung geeignete Bestandteile der Wirkstoffformulierung sind beispielsweise Lactose, Lactose Monohydrat, Glucose, Saccharose, Mannit, und Sorbit, vorzugsweise Lactose oder Lactose Monohydrat, insbesondere Lactose Monohydrat.

Ebenso bevorzugt ist ein Verfahren, worin die Pulverformulierung die Wirkstoffformulierung zur Inhalation ist. Pulver zur Inhalation können beispielsweise die Wirkstoffe ausgewählt aus der Gruppe bestehend aus Tiotropium, Cromoglicinsäure, Reproterol, Beclomethason, Terbutalin, Salbutamol, Salmeterol, Ketotifen, Orciprenalin Fluticason, Insulin, Ipratropium, Dexamethason, Bambuterol, Budesonid, Fenoterol, Clenbuterol Prednisolon, Prednison, Prednyliden, Methylprednisolon, Formoterol, Nedocromil, sowie eines ihrer pharmazeutisch verträglichen Salze oder Gemische und einem anderen für Inhalationszwecke geeigneten Kortisonpräparat oder Atropinderivat, bevorzugt Ipratropiumbromid, Tiotropiumbromid und Tiotropiumbromid Monohydrat, besonders bevorzugt Tiotropiumbromid Monohydrat, enthalten.

Typische Bestandteile von Pulvern zur Inhalation sind neben dem Wirkstoff u.a. Lactose, Lactose Monohydrat oder Glucose.

Insbesondere bevorzugt ist ein Verfahren, worin die Reinigung in geschlossenen Kapseln erfolgt.

Von besonderer Bedeutung ist ein Verfahren, worin die Reinigung ohne Lösungsmitteleinsatz erfolgt.

Für das erfindungsgemäße Verfahren geeignete Freifallmischer sind beispielsweise Rhönrad ELTE 650 oder Rhönrad SA 1200 (Fa. Engelsmann AG, Frankenthaler Straße 137 -141, D 6700 Ludwigshafen/Rh), Turbula T2 C (Fa. Bachofen AG, Basel, Schweiz) oder Turbula T 10 B (Fa. Bachofen AG, Basel, Schweiz), insbesondere geeignet Turbula T2 C oder Turbula T 10 B.

Weiterhin von besonderer Bedeutung ist ein Verfahren, worin die Reinigung bei einer Temperatur von 15 bis 50 °C, vorzugsweise 17 bis 40 °C, bevorzugt 19 bis 28 °C, insbesondere bevorzugt etwa 22 °C, erfolgt.

Weiterhin besonders bevorzugt ist ein Verfahren, worin ein Teil oder alle Körner der Pulverformulierung Verunreinigungen anlagern.

Ferner bevorzugt ist ein Verfahren, worin ein Teil oder alle Körner der Pulverformulierung Schmier- und Trennmittel anlagern. Solche Schmier- und Trennmittel können beispielsweise Stearinsäure, Magnesiumstearat, Fette, Wachse, Öle oder Emulgatoren wie Sojalecithin enthalten.

Ferner besonders bevorzugt ist ein Verfahren, worin die Füllmenge der Pulverformulierung von 6 % (v/v) bis 50 % (v/v), vorzugsweise 10 % (v/v) bis 30 % (v/v), bevorzugt 15 % (v/v) bis 25 % (v/v), besonders bevorzugt etwa 20 % (v/v), der maximalen Füllmenge der Kapsel beträgt.

Ferner insbesondere bevorzugt ist ein Verfahren, worin die Pulverformulierung Lactose und/oder Lactose Monohydrat, vorzugsweise Lactose Monohydrat für Inhalationszwecke, bevorzugt Lactose Monohydrat für Inhalationszwecke 200M enthält. Lactose Monohydrat ist beispielsweise erhältlich bei der Fa. DMV International (Veghel/ Niederlande).

Erfindungsgemäß bevorzugt ist ein Verfahren, worin die Pulverformulierung einen Hilfsstoff mit Partikelgrößen von 10 bis 50 µm aerodynamischem Durchmessers (gemessen mit einem API Aerosizer LD mittels einer Flugzeitmethode) enthält, beispielsweise gemahlenes Lactose Monohydrat.

Erfindungsgemäß besonders bevorzugt ist ein Verfahren, welches die aufeinanderfolgenden Schritte a) bis e) umfaßt, worin
a) eine Pulverformulierung einer oder mehrerer Siebungen und Mischungen unterworfen wird,
b) die Pulverformulierung in Gelatinekapseln zur Inhalation gefüllt wird,
c) die Gelatinekapseln in einem Mischbehälter bewegt werden,
d) das Ende des Reinigungsvorgangs, gegebenenfalls durch Öffnen von Kapseln, visuell überprüft wird und
e) die Gelatinekapseln direkt in den Inhalator eingesetzt oder gegebenenfalls entleert und neu befüllt werden.

Der Vorteil des erfindungsgemäßen Verfahrens liegt in der einfachen und ökonomischen Reinigung der Kapselinnenwand, die zudem keine zusätzliche Kontrolle des Gehalts an Reinigungsmittel z.B. Lösungsmittel erfordert. Im Falle von pharmakologisch verträglichen Verunreinigungen der Kapselinnenwandung kann die Reinigung durch die Wirkstoffformulierung erfolgen, so daß die Kapsel nach der Reinigung nicht nochmals geöffnet, entleert und erneut mit der Wirkstoffformulierung gefüllt werden muß. Der Reinigungsvorgang gewährleistet auch in diesem Fall eine Wirkstofffreigabe, die nicht durch Haftung der Formulierung an der Innenwand der Kapseln beeinträchtigt ist.

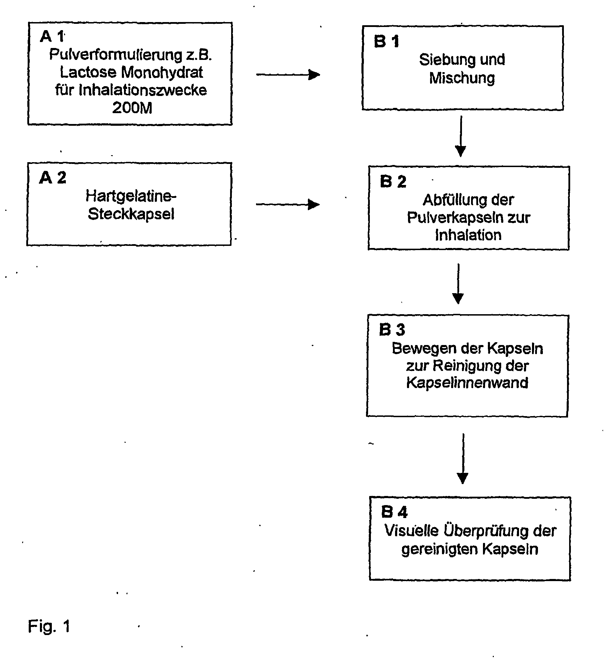

Das erfindungsgemäße Verfahren kann nach der im Flowchart der Figur 1 dargestellten allgemeinen Vorgehensweise und der im folgenden beschriebenen bevorzugten Ausführungsform durchgeführt werden. Sie sind als Erläuterung der Erfindung zu verstehen, ohne selbige auf deren Gegenstand zu beschränken.

Für die Reinigung der Gelatinekapseln mit einer Pulverformulierung nach dem erfindungsgemäßen Verfahren eignen sich insbesondere Pulverformulierungen, welche Lactose Monohydrat enthalten (A1), das zuvor gemischt und gesiebt wurde. Dazu wird Lactose Monohydrat über ein geeignetes Sieb (B1), beispielsweise ein Handsieb mit einer Maschenweite von 0,2 mm bis 1 mm, vorzugsweise etwa 0,5 mm, oder einen geeigneten Siebgranulator mit einer Maschenweite von 0,2 mm bis 1 mm, vorzugsweise ca 0,5 mm bis 0,6 mm, in einen Auffangbehälter- oder Mischbehälter gesiebt. Das Siebgut wird in einem geeigneten Mischer , beispielsweise in Rhönrad ELTE 650 oder Rhönrad SA_1200 (Fa. Engelsmann AG, Frankenthaler Straße 137 - 141, D 6700 Ludwigshafen/Rh), Turbula T2 C (Fa. Bachofen AG, Basel, Schweiz) oder Turbula T 10 B (Fa. Bachofen AG, Basel, Schweiz), homogen über eine Zeitraum von 20 bis 150 Minuten, vorzugsweise 50 bis 100 Minuten, bevorzugt 60 bis 90 Minuten, besonders bevorzugt etwa 30 bis 75 Minuten, mit vorzugsweise 5 bis 35, bevorzugt 10 bis 30, besonders bevorzugt etwa 20 Umdrehungen/Minute gemischt. Als geeigneter Siebgranulator ist beispielsweise QUADRO Comil, Typ: 197 S, 0,5 mm (Intertechnik Elze GmbH & Co KG, Lessingweg 1+2, D 31008 Elze) oder Glatt Schnellsieb, Typ TR 80, 0,6 mm (Fa. Glatt GmbH, D 7851 Binzen / Lörrach) einsetzbar.
Während der Siebung und Mischung sollte ein Raumklima mit einer Temperatur von 19°C bis 28 °C, vorzugsweise 22 °C, und einer relativen Luftfeuchtigkeit von 35 % r.F. bis 65 % r. F., vorzugsweise 50 % r. F. vorliegen.
Das gesiebte Lactose Monohydrat wird gesondert oder als Bestandteil einer Pulverformulierung mit einer geeigneten Kapselfüll- und -schließmaschine (B2), beispielsweise Typ MG2-G100 (Fa. MG2, Bologna, Italien) in Gelatinesteckkapseln (A2), vorzugsweise polyethylenglycolfreie Gelatinesteckkapseln, gefüllt. Die Füllmenge der Pulverformulierung sollte 6 % (v/v) bis 50 % (v/v), vorzugsweise 10 % (v/v) bis 30 % (v/v), bevorzugt 15 % (v/v) bis 25 % (v/v), besonders bevorzugt etwa 20 % (v/v), der maximalen Füllmenge der Kapsel betragen. Während der Abfüllung sollte ebenfalls das während der Siebung und Mischung bevorzugte Raumklima vorliegen.
Die abgefüllten und verschlossenen Kapseln werden unter oben beschriebenen Klimabedingungen in ein geeignetes Mischgefäß gegeben. Die Füllhöhe beträgt 50 % bis 80 % , vorzugsweise 60 % bis 70 %, insbesondere bevorzugt etwa 65 %, der Gefäßhöhe.
Das Mischgefäß wird anschließend in einem geeigneten Mischer (B3), vorzugsweise Rhönrad ELTE 650 oder Rhönrad SA_1200 (Engelsmann AG, Frankenthaler Straße 137 -141, D 6700 Ludwigshafen/Rh), Turbula T2 C, oder Turbula T 10 B (Fa Bachofen AG (Schweiz)mit 10 bis 30 U/min vorzugsweise mit etwa 20 U/min, bewegt. Die Mischzeit sollte 20 bis 150 Minuten, vorzugsweise 50 bis 100 Minuten, bevorzugt 60 bis 90 Minuten, besonders bevorzugt etwa 30 bis 75 Minuten betragen. Das Ergebnis des Mischungs- bzw. Reinigungsvorgangs kann über eine visuelle Kontrolle, gegebenenfalls durch Öffnen von Kapseln, geprüft werden (B4). Der Reinigungsvorgang ist beendet, sobald keine Pulverbelegung der Kapselinnenwand mehr zu beobachten ist. Auch eine lediglich geringfügige Pulverbelegung der Kapselinnenwand ist für die Beendigung des Reinigungsvorgangs ausreichend.
Die Kapseln können entleert und anschließend mit einer Wirkstoffformulierung gefüllt werden. Wurde als Pulverformulierung die Wirkstoffformulierung selbst eingesetzt, so ist keine Entleerung und Neubefüllung der Kapseln erforderlich. Sie können direkt für eine Inhalation verwendet werden.

Das folgende Beispiel dient der näheren Erläuterung des erfindungsgemäßen Verfahrens. Es ist lediglich als exemplarische Vorgehensweise zu verstehen, ohne die Erfindung auf deren Inhalt zu beschränken.

### Beispiel

Es wurden jeweils etwa 70 Hartgelatinekapseln der Qualität A (reduzierter Gehalt an Schmier- und Trennmittel), Typ CONI-SNAP® (Fa.Capsugel) Größe 3 und der Qualität B (Standard), Typ CONI-SNAP® (Fa.Capsugel) Größe 3 mit einer Tiotropium-Lactose-Mischung aus ca. 11 mg/Kapsel Lactose Monohydrat (Pharmatose 200M der Fa. Veghel / NL) und 36 µg/Kapsel Tiotropiumbromid gefüllt. Die Hälfte der Kapseln wurde mit einem Turbula Mischer Typ 2C (Fa. Bachofen AG, Basel, Schweiz) für etwa zwei Stunden bei einer Temperatur von 22 °C geschüttelt. Anschließend wurden der Parameter "Ausgebrachte Dosis", d.h. ausgebrachter Wirkstoffanteil in %, in den geschüttelten und ungeschüttelten Kapseln bestimmt.

Die Werte der Tabelle 1 zeigen einen deutlichen Anstieg der ausgebrachten Dosis (bestimmt nach TEST Uniformity of dose, a. Uniformity of delivered dose, European PharmacopeiaThird Edition (1997) page 1770, published by the Council of Europe, 67075 Strasbourg Cedex, ISBN: 92-871-2991-6) nach dem Reinigungsvorgang durch Schütteln der Kapseln.

**Tabelle 1**

| Kapsel | Ausgebrachte Dosis [%] |
|---|---|
| Qualität A ungeschüttelt | 74 |
| Qualität A geschüttelt | 82 |
| Qualität B ungeschüttelt | 79 |
| Qualität B geschüttelt | 83 |

## Patentansprüche

1. Verfahren zur Reinigung der Innenwand von Hartgelatinekapseln zur Verwendung in der Inhalationstherapie, **dadurch gekennzeichnet, daß** die Kapseln durch Schütteln mit einer Pulverformulierung gereinigt werden und **daß** die Reinigung in einem Freifallmischer oder auf einem Rütteltisch erfolgt.

2. Verfahren nach Anspruch 1, **dadurch gekennzeichnet, daß** die Pulverformulierung pharmazeutisch verträglich ist

3. Verfahren nach einem der Ansprüche 1 oder 2, **dadurch gekennzeichnet, daß** die Pulverformulierung einen Bestandteil der Wirkstoffformulierung darstellt

4. Verfahren nach einem der Ansprüche 1 oder 2, **dadurch gekennzeichnet, daß** die Pulverformulierung die Wirkstoffformulierung zur Inhalation ist.

5. Verfahren nach einem der Ansprüche 1 bis 4, **dadurch gekennzeichnet, daß** die die Reinigung in geschlossenen Kapseln erfolgt.

6. Verfahren nach einem der Ansprüche 1 bis 5, **dadurch gekennzeichnet, daß** die Reinigung ohne Lösungsmitteleinsatz erfolgt.

7. Verfahren nach einem der Ansprüche 1 bis 6, **dadurch gekennzeichnet, daß** die Reinigung bei einer Temperatur von 15 bis 50 °C erfolgt.

8. Verfahren nach einem der Ansprüche 1 bis 7, **dadurch gekennzeichnet, daß** ein Teil oder alle Körner der Pulverformulierung Verunreinigungen anlagern.

9. Verfahren nach einem der Ansprüche 1 bis 8, **dadurch gekennzeichnet, daß** ein Teil oder alle Körner der Pulverformulierung Schmier- oder/und Trennmittel anlagern.

10. Verfahren nach einem der Ansprüche 1 bis 9, **dadurch gekennzeichnet, daß** die Füllmenge der Pulverformulierung von 6 % bis 50 % der theoretischen Gesamtfüllmenge der Kapsel beträgt.

11. Verfahren nach einem der Ansprüche 1 bis 10, **dadurch gekennzeichnet, daß** die Pulverformulierung mindestens einen Hilfsstoff mit Partikelgrößen von 10 bis 50 µm aerodynamischem Durchmessers enthält.

12. Verfahren nach einem der Ansprüche 1 bis 11, **dadurch gekennzeichnet, daß** die Pulverformulierung Lactose und/oder Lactose Monohydrat enthält.

13. Verfahren nach einem der Ansprüche 1 bis 12, welches die aufeinanderfolgenden Schritte a) bis e) umfaßt, worin
a) eine Pulverformulierung einer oder mehrerer Siebungen und Mischungen unterworfen wird,
b) die Pulverformulierung in Gelatinekapseln zur Inhalation gefüllt wird,
c) die Gelatinekapseln in einem Mischbehälter bewegt werden,
d) das Ende des Reinigungsvorgangs visuell überprüft wird und
e) die Gelatinekapseln direkt in den Inhalator eingesetzt oder gegebenenfalls entleert und neu mit der Wirkstoffformulierung befüllt werden.

## Claims

1. Process for cleaning the inner wall of hard gelatine capsules for use in inhalation therapy, **characterised in that** the capsules are cleaned by shaking with a powder formulation and **in that** the cleaning is carried out in a gravity mixer or on a vibrating table.

2. Process according to claim 1, **characterised in that** the powder formulation is pharmaceutically acceptable.

3. Process according to one of claims 1 or 2, **characterised in that** the powder formulation constitutes an ingredient of the active substance formulation.

4. Process according to one of claims 1 or 2, **characterised in that** the powder formulation is the active substance formulation for inhalation.

5. Process according to one of claims 1 to 4, **characterised in that** the cleaning is carried out in sealed capsules.

6. Process according to one of claims 1 to 5, **characterised in that** the cleaning is carried out without the use of solvents.

7. Process according to one of claims 1 to 6, **characterised in that** the cleaning is carried out at a temperature of 15 to 50 °C.

8. Process according to one of claims 1 to 7, **characterised in that** some or all of the grains of the powder formulation accumulate impurities.

9. Process according to one of claims 1 to 8, **characterised in that** some or all of the grains of the powder formulation accumulate lubricants and/or mould release agents.

10. Process according to one of claims 1 to 9, **characterised in that** the fill level of powder formulation is from 6 % to 50 % of the theoretical total capacity of the capsule.

11. Process according to one of claims 1 to 10, **characterised in that** the powder formulation contains at least one excipient with particle sizes of 10 to 50 µm in aerodynamic diameter.

12. Process according to one of claims 1 to 11, **characterised in that** the powder formulation contains lactose and/or lactose monohydrate.

13. Process according to one of claims 1 to 12, which comprises the successive steps a) to e), wherein
a) a powder formulation is subjected to one or more screenings and mixings,
b) the powder formulation is transferred into gelatine capsules for inhalation,
c) the gelatine capsules are agitated in a mixing container,
d) the end of the cleaning process is monitored visually, and
e) the gelatine capsules are placed directly in the inhaler or optionally emptied and refilled with the active substance formulation.

## Revendications

1. Procédé servant à nettoyer la paroi intérieure de capsules de gélatine dure destinées à être utilisées lors de la thérapie par inhalation, **caractérisé en ce que** les capsules sont nettoyées en les secouant avec une formulation de poudre, et que le nettoyage est effectué dans un malaxeur à chute libre ou sur une table vibrante.

2. Procédé selon la revendication 1, **caractérisé en ce que** la formulation de poudre est pharmaceutiquement acceptable.

3. Procédé selon l'une quelconque des revendications 1 ou 2, **caractérisé en ce que** la formulation de poudre constitue une composante de la formulation de principes actifs.

4. Procédé selon l'une quelconque des revendications 1 ou 2, **caractérisé en ce que** la formulation de poudre est la formulation de principes actifs destinée à l'inhalation.

5. Procédé selon l'une quelconque des revendications 1 à 4, **caractérisé en ce que** le nettoyage est effectué dans des capsules fermées.

6. Procédé selon l'une quelconque des revendications 1 à 5, **caractérisé en ce que** le nettoyage est effectué sans l'emploi de solvant.

7. Procédé selon l'une quelconque des revendications 1 à 6, **caractérisé en ce que** le nettoyage est effectué à une température allant de 15 à 50 °C.

8. Procédé selon l'une quelconque des revendications 1 à 7, **caractérisé en ce qu'**une partie ou tous les grains de la formulation de poudre accumulent des impuretés.

9. Procédé selon l'une quelconque des revendications 1 à 8, **caractérisé en ce qu'**une partie ou tous les grains de la formulation de poudre accumulent des agents de lubrification et/ou de séparation.

10. Procédé selon l'une quelconque des revendications 1 à 9, **caractérisé en ce que** la quantité de remplissage de la formulation de poudre va de 6 % à 50 % de la quantité de remplissage totale théorique des capsules.

11. Procédé selon l'une quelconque des revendications 1 à 10, **caractérisé en ce que** la formulation de poudre contient au moins un adjuvant avec des tailles de particules ayant un diamètre aérodynamique allant de 10 à 50 µm.

12. Procédé selon l'une quelconque des revendications 1 à 11, **caractérisé en ce que** la formulation de poudre contient du lactose et/ou du monohydrate de lactose.

13. Procédé selon l'une quelconque des revendications 1 à 12, qui comprend les étapes a) à e) se suivant les unes les autres, dans lequel
a) une formulation de poudre est soumise à un ou à plusieurs criblages et mélanges,
b) la formulation de poudre est remplie dans des capsules de gélatine aux fins de l'inhalation,
c) les capsules de gélatine sont déplacées dans un contenant mélangeur,
d) la fin de l'opération de nettoyage est vérifiée visuellement, et
e) les capsules de gélatine sont directement insérées dans l'inhalateur ou sont éventuellement vidées et sont à nouveau remplies avec la formulation de principes actifs.
